# EUROPEAN PATENT APPLICATION

(11) **EP 0 912 075 A1**
(43) Date of publication of application: **28.04.1999**
(21) Application number: 97203295.7
(22) Date of filing: 24.10.1997
(51) Int. Cl.: H04R 29/00

(54) **Method for testing the sound attenuation of hearing protecting means mounted in helmets**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: The designation of the inventor has not yet been filed
(74) Representative: Smulders, Theodorus A.H.J., Ir.

(57) **Abstract**

A method for testing the sound attenuation of hearing protecting means mounted in helmets, said helmets comprising at least an earphone, said method comprising the steps of: a) placing the helmet to be tested in a test position; b) switching the earphone in such a manner that it acts as a sound pressure sensor or microphone; c) generating at least a sound pressure outside the helmet; detecting electric signals which are representative of said generated sound pressure and are received by said earphone; and d) deriving from said electric signals information on acoustic leaks in the helmet.

## Description

The present invention relates to a method for testing the sound attenuation of hearing protecting means mounted in helmets, e.g. flighthelmets or racecar driver helmets and the like.

Generally, on board aircrafts such as fighter jets and helicopters or on board racecars and the like a lot of noise is generated. In order to protect acoustically the crew's ears, helmets are worn which are acoustically insulated and are provided with hearing protecting means. Such helmets may possess good noise attenuation qualities, but maximum attenuation may not always be realized, when the helmet is worn, particularly if the helmet does not fit in a proper manner. In that case, acoustic leaks may occur which have a negative effect on the protection of the bearer's hearing organ (the so-called noise dose) and on the speech communication between the bearer and the outside world, said speech communication generally taking place through at least an earphone which is mounted in the helmet in any way suitable for the purpose. Generally, such an earphone is connected through an audiocable to appropriate equipment.

Therefore, there is a need to provide a simple and cheap method for testing the sound attenuation of hearing protecting means mounted in helmets and it is an object of the present invention to provide such a testing method.

The invention therefore provides a method for testing the sound attenuation of hearing protecting means mounted in helmets, said helmets comprising at least an ear phone, said method comprising the steps of:
a) placing the helmet to be tested in a test position;
b) switching the earphone in such a manner that it acts as a sound pressure sensor or microphone;
c) generating at least a sound pressure outside the helmet; detecting electric signals which are representative for said generated sound pressure and are received by said earphone; and
d) deriving from said electric signals information on acoustic leaks in the helmet.

In particular, the invention provides a testing method enabling the verification of the proper helmet fitting in a cheap and simple manner. More in particular, there is no need to modify the helmet when using the method of the present invention.

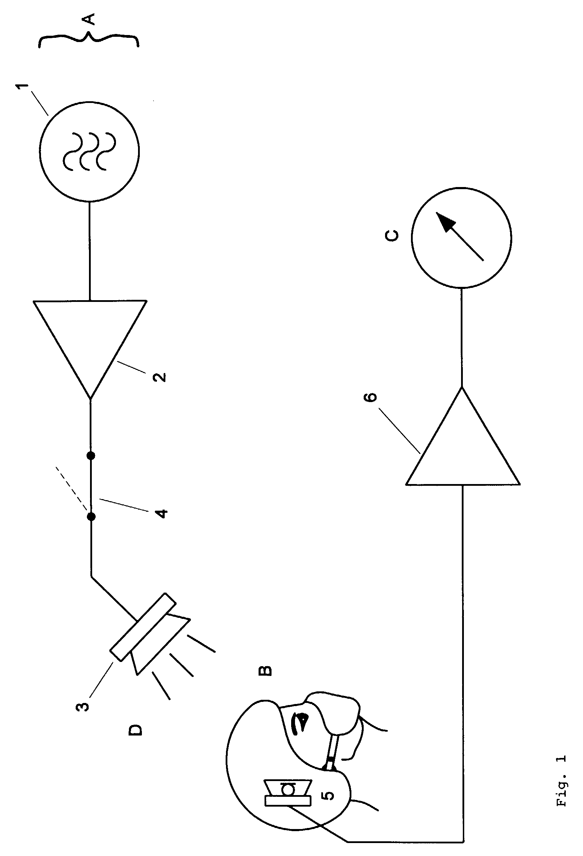

The present invention will now be described in more detail by way of example with reference to the accompanying drawing, in which the figure represents schematically the test principle of the present invention.

Referring now to the figure, a signal source (A) is shown, which generates acoustic noise outside a helmet (B) placed on the head of a test person.

The signal source (A) comprises a noise source (1), which is electrically connected in any suitable manner via an amplifier (2) and a switch (4) to a suitable loudspeaker (3) emitting sound pressure waves (D). It will be appreciated by those skilled in the art that any signal source suitable for the purpose may be applied. Generally, it will be appreciated by those skilled in the art, that the frequency spectrum covered by the acoustic signal comprises low-frequency components.

The earphone(s) (5) of the helmet (B) are connected electrically in any suitable manner through an amplifier (6) to a level indicator (C) using the electric output of the earphone as measurement of acoustic noise level. Advantageously, the signal level indicator is effective in a predetermined frequency band.

The invention has been based upon the understanding that a test for acoustic leakage in helmets can be carried out using the earphone (5) mounted in the helmet as a sound pressure sensor or microphone rather than a sound reproducing means without any modification of the helmet.

The method of the invention is carried out as follows:
1) Prior to testing, the helmet is placed on the bearer's head in a conventional manner. Generally, an assistant places the helmet on the bearer's head.
2) The audio cable of the helmet is connected in any suitable manner to a measuring device and in this manner the earphone(s) mounted in the helmet are connected to a signal level indicator.
3) Subsequently, a sound pressure, advantageously a broadband sound pressure, is generated outside the helmet.
4) This sound pressure is converted by the earphone(s) into an electric signal and the signal level will be indicated by the signal level indicator.

The signal level is inter alia dependent on the acoustic insulation of the earphone(s). If an acoustic leak would occur, e.g. by misfitting of the helmet on the bearer's head, this will be indicated by a change in signal level and in that case appropriate measures can be taken to provide a proper fitting of the helmet on the bearer's head.

In an advantageous embodiment of the present invention, the helmet is tested first in an open position (i.e. when not placed on the bearer's head) and the helmet is subsequently tested when placed on the bearer's head.

The difference between these two measurements provides a fair estimate of the sound attenuation. In this manner the method is made independent of other factors which may influence the signal level (e.g. sound pressure source, earphone sensitivity and the like). It will be appreciated by those skilled in the art that the frequency range of the system is determined by the choice of the sound spectrum applied.

Various modifications of the present invention will become apparent to those skilled in the art from the foregoing description. Such modifications are intended to fall within the scope of the appended claims.

## Claims

1. A method for testing the sound attenuation of hearing protecting means mounted in helmets, said helmets comprising at least an earphone, said method comprising the steps of:
a) placing the helmet to be tested in a test position;
b) switching the earphone in such a manner that it acts as a sound pressure sensor or microphone;
c) generating at least a sound pressure outside the helmet; detecting electric signals which are representative of said generated sound pressure and are received by said earphone; and
d) deriving from said electric signals information on acoustic leaks in the helmet.

2. The method as claimed in claim 1, wherein the helmet is placed on the bearer's head.

3. The method as claimed in claim 1, wherein the helmet is not placed on the bearer's head (i.e. testing is carried out with an open helmet).

4. The method as claimed in any one of claims 1-3, wherein the electric signals are recorded on a signal level indicator.

5. The method as claimed in any one of claims 1-4, wherein the sound pressure generated outside the helmet is a broadband sound.

6. The method as claimed in any one of claims 1-5, wherein the electric signals received by the earphone are outside a predetermined signal level, thus indicating the presence of acoustic leaks.

7. The method as claimed in any one of claims 1-6, comprising the steps of first testing the helmet in an open position and subsequently testing the helmet when placed on the bearer's head.

8. The method as claimed in claim 7, wherein the sound attenuation is derived from the difference of the signal measurements of said first test and said subsequent test.

9. The method as claimed in any one of claims 4-8, wherein the said signal level indicator is effective in a predetermined frequency band.
